(19) Europäisches Patentamt
European Patent Office
Office européen des brevets

(11) Veröffentlichungsnummer: **0 072 987**
**A1**

# (12) EUROPÄISCHE PATENTANMELDUNG

(21) Anmeldenummer: **82107361.6**

(22) Anmeldetag: **13.08.82**

(51) Int. Cl.³: **C 07 F 9/65**
**A 61 K 31/675**

(30) Priorität: **21.08.81 DE 3133077**

(43) Veröffentlichungstag der Anmeldung:
**02.03.83 Patentblatt 83/9**

(84) Benannte Vertragsstaaten:
**AT BE CH DE FR GB IT LI LU NL SE**

(71) Anmelder: **Henkel Kommanditgesellschaft auf Aktien**
**Postfach 1100 Henkelstrasse 67**
**D-4000 Düsseldorf 1(DE)**

(72) Erfinder: **Wilk, Hans-Christoph, Dr.**
**An der Obererft 94**
**D-4040 Neuss(DE)**

(72) Erfinder: **Möller, Hinrich, Dr.**
**Schumannstrasse 11**
**D-4019 Monheim(DE)**

(72) Erfinder: **Fischer, Herbert, Dr.**
**Am Nettchesfeld 14**
**D-4000 Düsseldorf 13(DE)**

(72) Erfinder: **Zeidler, Ulrich, Dr.**
**Heinrich-Lersch-Strasse 19**
**D-4000 Düsseldorf 13(DE)**

(72) Erfinder: **Hase, geb. Kornrumpf, Brigitte, Dr.**
**Millrather Weg 29**
**D-4006 Erkrath 1(DE)**

(54) **Neue cytostatisch wirksame Epoxidgruppen tragende 1,3,2-Oxazaphosphorin-Verbindungen, Verfahren zu ihrer Herstellung sowie ihre Verwendung in pharmazeutischen Zubereitungen.**

(57) Neue Glycidyl-substituierte 1,3,2-Oxazaphosphorin-Verbindungen der allgmeinen Formel I

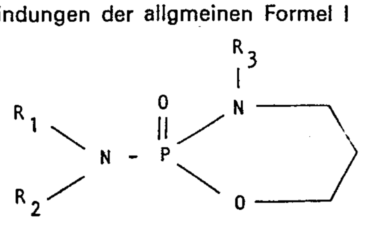

1

in $R_1$, $R_2$ und $R_3$ Wasserstoff, den Glycidylrest und/oder einen 3-Hydroxy-2-Halogen-Propylrest mit der Maßgabe bedeuten, daß wenigstens ein Glycidylrest zusammen mit wenigstens einem 3-Hydroxy-2-Halogen-Propyl-Rest vorliegen, wobei $R_1$ auch einen Kohlenwaserstoffrest bedeuten kann und Verfahren zur Herstellung dieser neuen Verbindungen wobei man in 1,3,2-Oxazaphosphorin-Verbindungen der allgmeinen Formel II

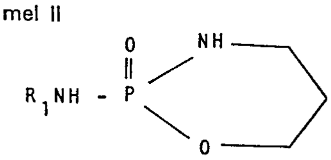

11

in der $R_1$ Wasserstoff oder einen Kohlenwasserstoffrest bedeutet, den Glycidylrest in bekannter Weise, einführt sowie Arzneimittelzubereitungen mit cytostatischer Wirksamkeit enthaltend Verbindungen der allgemeinen Formel I.

EP 0 072 987 A1

0072987

Henkelstraße 67
4000 Düsseldorf, den 27. April 1982

HENKEL KGaA
ZR-FE/Patente
HF/Br

# P a t e n t a n m e l d u n g

D 6413 EP

## Neue cytostatisch wirksame Epoxidgruppen tragende 1,3,2-Oxazaphosphorin-Verbindungen, Verfahren zu ihrer Herstellung sowie ihre Verwendung in pharmazeutischen Zubereitungen

Gegenstand der DE-OS 29 07 349 sind Arzneimittelzubereitungen mit cytostatischer Wirksamkeit, die als Wirkstoff Triglycidylisocyanurat (TGI) enthalten. Die drei N-Atome des Isocyanursäureringes sind hier mit Epoxidgruppen besetzt. Vermutlich ist die alkylierende Wirkung dieser Epoxidgruppen für die cytostatische Wirksamkeit des TGI-Derivats verantwortlich zu machen.

Die Europäische Patentanmeldung 81 100 544.6 schildert Arzneimittelzubereitungen mit cytostatischer Wirksamkeit, die durch einen Gehalt an N-Heterocyclen gekennzeichnet sind, welche wenigstens zwei Glycidyl-substituierte N-Atome in Amid-und/oder Imidform in das Ringsystem eingebunden, enthalten. Es werden zahlreiche spezielle Beispiele für solche N-Glycidyl-substituierte heterocyclische Verbindungen angegeben.

Bekannt ist weiterhin, daß eine ganz andere Stoffklasse ausgeprägte cytostatische Wirksamkeit aufweist. Es handelt sich hier um Verbindungen aus der Klasse der substituierten 1,3,2-Oxazaphosphorin-Verbindungen. So beschreibt die GB-PS 812 651 das 2 H-1,3,2-Oxazaphosphorin-2-amin-N,N-bis-(2-chloroethyl-) tetrahydro-2-oxid, während in der FR-PS 1 530 962 das 2 H-1,3,2-Oxazaphosphorin-3(2-chlorethyl)-2-[(2-chlorethylamino]-

...

Sd 230 3. 08.80

tetrahydro-2-oxid beschrieben ist. Bekannt ist auch das entsprechende Derivat mit drei 2-Chlorethylgruppen.

Es wurde jetzt im Tierversuch festgestellt, daß neue Glycidyl-substituierte 1,3,2-Oxazaphosphorin-Verbindungen der im folgenden angegebenen Struktur ausgesprochene cytostatische Aktivität aufweisen.

Gegenstand der Erfindung sind dementsprechend in einer ersten Ausführungsform neue Glycidyl-substituierte 1,3,2-Oxazaphosphorin-Verbindungen der allgemeinen Formel I

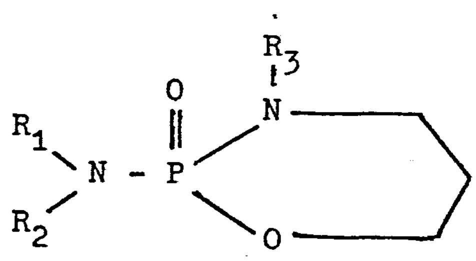

I

in der $R_1$, $R_2$ und $R_3$ Wasserstoff, den Glycidylrest und/oder einen 3-Hydroxy-2-Halogen-Propylrest mit der Maßgabe bedeuten, daß wenigstens 2 Glycidylreste oder wenigstens 1 Glycidylrest zusammen mit wenigstens einem 3-Hydroxy-2-Halogen-Propylrest vorliegen, wobei $R_1$ auch einen Kohlenwasserstoffrest bedeuten kann.

Die Erfindung betrifft in einer weiteren Ausführungsform Arzneimittelzubereitungen, die durch einen Gehalt an den neuen Glycidyl-substituierten 1,3,2-Oxazaphosphorin-Verbindungen der allgemeinen Formel I gekennzeichnet sind und diese insbesondere in Abmischung mit üblichen Hilfs- beziehungsweise Trägerstoffen für pharmakologische Zubereitungen enthalten. Schließlich betrifft die Erfindung in einer weiteren Ausführungsform das Verfahren zur Herstellung der neuen cytostatisch aktiven Verbindungen der allgemeinen Formel I.

...

Der Wirkungsmechanismus der im Rahmen der Erfindung eingesetzten Verbindungen ist im einzelnen nicht geklärt. Vermutlich sind jedoch auch hier - wie bei den Glycidyl-substituierten Verbindungen der DE-OS 29 07 349 beziehungsweise der Europäischen Patentanmeldung 81 100 544.6 die Glycidylreste für die cytostatische Wirkung mitverantwortlich. Neben der erfindungsgemäß wenigstens einfach vorliegenden Glycidylgruppe dürfte auch dem 3-Hydroxy-2-chloro-propyl-Rest Bedeutung zukommen.

In einer bevorzugten Ausführungsform der Erfindung liegt einer dieser aktiven Reste am Ringstickstoff als Rest $R_3$ vor. Die beiden verbleibenden Reste $R_1$ und $R_2$ oder wenigstens einer dieser beiden Reste sind gleichfalls ein Glycidyl-Rest oder der genannte 3-Hydroxy-2-Halogenpropyl-Rest.

In den Rahmen der Erfindung fallen allerdings auch solche Oxazaphosphorin-Verbindungen, in denen insgesamt nur 2 der genannten aktiven Substituenten in N-Stellung vorliegen. Insbesondere kann hier der Rest $R_1$ ein Kohlenwasserstoffrest sein, der auch Heteroatome enthalten kann. Als Heteroatome kommen insbesondere N, O, S und/oder P in Betracht. Bevorzugt enthält dieser Rest insgesamt nicht mehr als 12 Kohlenstoffatome und dabei zweckmäßig nicht mehr als 8 Kohlenstoffatome. Interessant können insbesondere Reste sein, die bis zu 6 oder vorzugsweise sogar nur bis zu 4 Kohlenstoffatome enthalten, wobei die Zahlenwerte unabhängig von der jeweiligen Struktur zu verstehen sind und sich lediglich auf die Summe aller Kohlenstoffatome im betroffenen Rest beziehen.

...

Bedeutet R einen Aryl-, Aralkyl- oder Alkarylrest, so sind hier insbesondere 1-kernige Substituenten bevorzugt. Typische Vertreter sind Phenyl, Benzyl, Tolyl, Xylyl und verwandte Verbindungen. Auch bei den cycloaliphatischen Ringen für den Rest $R_1$ sind 1-kernige Ringsysteme auf der Basis von Cyclopentyl, Cyclohexyl und ihren Abkömmlingen bevorzugt. Entsprechende heterocyclische Reste, insbesondere also 1-kernige Ringverbindungen mit O, N und/oder S im System fallen in den Rahmen der Erfindung. Die Ringsysteme können dabei bevorzugt 1,2 oder 3 solcher Heteroatome enthalten. Diese heterocyclischen Reste enthalten vorzugsweise 5 oder 6 Ringglieder.

In einer bevorzugten Ausführungsform der Erfindung bedeutet im Falle $R_1$ = Kohlenwasserstoffrest dieser Rest einen Alkylrest, der auch substituiert sein kann. Dieser Alkylrest kann geradkettig oder verzweigt und gesättigt oder ungesättigt sein und enthält vorzugsweise nicht mehr als 8 insbesondere nicht mehr als 6 Kohlenstoffatome. Besonders bevorzugt sind in dieser Ausführungsform unsubstituierte Alkylreste mit bis zu 4 C-Atome.

Liegen an dem Rest $R_1$ Substituenten vor, so gilt hier für die im Rahmen der Erfindung bevorzugt eingesetzten Verbindungen der allgemeinen Formel I die Regel, daß solche substituierenden Gruppen wenigstens unter Normalbedingungen keine oder keine wesentliche Reaktivität mit der Epoxidgruppe des Glycidylsubstituenten zeigen. Auf diese Weise ist sichergestellt, daß die erfindungsgemäßen Verbindungen hinreichend lagerbeständig sind und keine unerwünschte Umsetzung unter Vernichtung der Epoxidgruppierungen stattfindet.

...

Das bevorzugte Halogen im 3-Hydroxy-2-Halogen-Propylrest für $R_1$, $R_2$ und/oder $R_3$ ist Chlor, wenn auch die anderen Halogene insbesondere Brom nicht ausgeschlossen sind.

In einer weiteren Ausführungsform betrifft die Erfindung das Verfahren zur Herstellung der neuen 1,3,2-Oxazaphosphorin-Verbindungen der allgemeinen Formel I. Das Verfahren ist dadurch gekennzeichnet, daß man in 1,3,2-Oxazaphosphorin-Verbindungen der allgemeinen Formel II den Glycidylrest und/oder den 3-Hydroxy-2-Halogen-propylrest in an sich bekannter Weise einführt. In dieser allgemeinen Formel II bedeutet $R_1$ Wasserstoff oder einen Kohlenwasserstoffrest der zuvor angegebenen Konstitution. Geeignet ist zur Herstellung der neuen Verbindungen der allgemeinen Formel I insbesondere die Umsetzung entsprechender Phosphorsäureesterdiamide mit Epihalohydrinen und anschließender vollständiger oder wenigstens teilweise Abspaltung von Halogenwasserstoff mit Alkali.

Die Herstellung der 1,3,2-Oxazaphosphorin-Verbindungen der allgemeinen Formel II kann nach literaturbekannten Verfahren erfolgen. Zur einschlägigen Literatur wird beispielsweise verwiesen auf:
K. Sasse in Houben Weyl: "Methoden der Organischen Chemie" Band 12/2, S. 407 (Verlag Thome 1964).

Die Umsetzung dieser Ausgangsverbindungen der allgemeinen Formel II mit Epihalogenhydrinen erfolgt in an sich bekannter Weise. Sie kann in Gegenwart geringer Mengen an Basen, beispielsweise tertiärer Amine oder auch quartärer Ammoniumverbindungen, als Katalysator erfolgen. Die Umsetzung erfolgt zweckmäßigerweise im Temperatur-

...

bereich bis etwa 150 °C. Die bevorzugten Epihalohydrine enthalten Chlor oder gegebenenfalls Brom als Halogen. Die Reaktion kann gewünschtenfalls in geeigneten Lösungsmitteln insbesondere aprotischen Lösungsmitteln vorgenommen werden, die wenigstens einen der Reaktionspartner lösen und den Reaktanten gegenüber nicht reaktiv sind. Die bevorzugte Reaktionszeit beträgt 1 bis 10 Stunden, insbesondere 2 bis 5 Stunden. Die erfindungsgemäßen Verbindungen der allgemeinen Formel I können aus den Reaktionsrohgemischen in konventioneller Weise, zum Beispiel durch chromatographische Trennverfahren, abgetrennt und in reiner Form gewonnen werden. Bevorzugt werden diese neuen Verbindungen der allgemeinen Formel I in einer solchen Form, insbesondere in solcher Reinheit gewonnen, die ihre Verwendung als Arzneimittel zuläßt.

Die erfindungsgemäßen Arzneimittelmischungen, die sich durch einen Gehalt an Verbindungen der allgemeinen Formel I kennzeichnen, enthalten diese Wirksubstanzen in geeigneten Vehikeln. Hier eignen sich die üblichen Hilfs- beziehungsweise Trägerstoffe für pharmakologische Zubereitungen. Gegenstand der Erfindung ist schließlich die Verwendung dieser Verbindungen beziehungsweise Stoffgemische zur Therapie maligner Neoplasien. Die erfindungsgemäß eingesetzten Verbindungen sind wirksam gegen verschiedene Leukämieformen wie maligne Neoplasmen, wie Lungenkarzinom, Colonkarzinom, Melanome, Ependymoblastom und Sarcome. Eine Kombinationstherapie in Verbindung mit anderen Cytostatika, wie Derivaten des Stickstofflosts oder auch Fluoruracil ist möglich.

...

## Beispiele

1. Herstellung von 2H-1,3,2-Oxazaphosphorin, 2-(bis-glycidyl)aminotetrahydro-2-oxid-3-glycidyl-

10 g 2H-1,3,2-Oxazaphosphorin, 2-aminotetrahydro-2-oxid (0,073 mol) werden mit 275 ml Epichlorhydrin und 1 ml Triethylamin 4 h unter Rückfluß zum Sieden erhitzt. Man kühlt auf 40 °C, fügt 18 g 50 %ige NaOH und 50 g trockenes Molekularsieb 4 Å zu, rührt 1 h bei dieser Temperatur, filtriert ab und engt ein. Das Rohprodukt (ca. 20 g) wird durch Säulenchromatographie vorgereinigt: feste Phase Kieselgel 60 (Merck) Laufmittel Aceton, Laufmittelmenge dreifaches Volumen der 25 cm langen Säule. Nach Einengen i. Vac. wird das so vorgereinigte Produkt durch fraktionierte Säulenchromatographie in Aceton/Methylenchlorid (1:1) von den Chlorhydrinen, die einen höheren RF-Wert haben, abgetrennt. Ausbeute 2,63 g (12 %) Epoxidsauerstoff: 16,4 % (theor.: 15,9 %). Das Produkt kristallisierte innerhalb von 3 Tagen bei 4 °C. Massenspektrum IR und 'H-NMR stützen die Struktur.

2. Herstellung von 2H-1,3,2-Oxazaphosphorin, 2-(glycidylmethyl)amino-2-oxid-3-(3-hydroxy-2-chloro)-propyl-tetrahydro -

10 g 2H-1,3,2-Oxazaphosphorin, 2-methylaminotetrahydro-2-oxid (0,067 mol) wurden wie vorstehend beschrieben mit Epichlorhydrin umgesetzt und durch Säulenchromatographie (Aceton) vorgereinigt (zweimal). Die Rohausbeute betrug 9,4 g. Die Dünnschichtchromatographie des Rohproduktes (feste Phase Kieselgel 60 (Merck) Laufmittel Aceton) zeigte drei Produkte

...

mit $R_f$-Werten zwischen 0,66 und 0,78. (Sichtbar gemacht durch Bedampfen mit Jod). Durch Säulenchromatographie wird das Produkt mit $R_f$-Wert 0,66 (Aceton) isoliert. Ausbeute: 2,7 g (14 % d.Th.), 6,1 % Epoxidsauerstoff (theor. 5,5), 11,4 % Cl (theor. 12,3). IR- und H-NMR- und Massenspektrum stützen die Struktur.

3. Herstellung von 2H-1,3,2-Oxazaphosphorin, 2-[glycidyl-(3-hydroxy-2-chloro-propyl)]-amino-tetrahydro-2-oxid-3-methyl -

10 g 2H-1,3,2-Oxazaphosphorin-2-amino-3-methyl-tetrahydro-2-oxid (0,067 mol) wurden in der beschriebenen Weise mit Epichlorhydrin umbesetzt. Nach zweimaligem Vorreinigen durch Säulenchromatographie wurden 18 g Rohprodukt erhalten. Es wurden die in Petrolether unlösliche Bestandteile abgetrennt und durch Säulenchromatographie (Aceton) ein Produkt mit $R_f$ = 0,62 (Hauptbestandteil) isoliert. Ausbeute 3,4 g (18 % d. Th.), 4,7 % Epoxidsauerstoff (theor. 5,5), 11,9 % Chlor (theor. 12,3). IR,- H-NMR- und Massenspektrum stützen die Struktur.

4. Mit den isolierten Wirksubstanzen der Beispiele 1 bis 3 werden die nachfolgenden Versuche durchgeführt nach den Testvorschriften des National Cancer Institutes Bethesda, Maryland 20014, veröffentlicht in "Cancer Chemotherapy Reports" Part 3, September 1972, Vol. 3 No. 2. Die Substanz wurde jeweils als wäßrige 1 %ige Injektionslösung unmittelbar vor der Applikation frisch hergestellt.

Bei Mäusen wurde gemäß Protokoll 1200 (Seite 91 c) die Tumorart PS 2684 i.p. mit $10^6$ Zellen/Maus gesetzt. Die mittlere Überlebensdauer der unbehandelten Tiere wird bestimmt. ...

In weiteren Versuchsgruppen wird den behandelten Tieren der Wirkstoff appliziert. In unterschiedlichen Versuchsreihen werden dabei als Einzelgaben an 9 Tagen Injektionen in wechselnden im folgenden angegebenen Einzelmengen verabreicht. In allen Fällen wird eine signifikante Verlängerung der Lebensdauer der behandelten Testtiere gegenüber der mittleren Überlebensdauer der nicht mit dem Wirkstoff behandelten Tiere erzielt. Die Verlängerungsrate T/C in Abhängigkeit von der Dosierung des Wirkstoffs ist wie folgt:

| Verabreichte Dosis mg/kg | T/C-Wert |
|---|---|
| 6,25 | 158 |
| 3,12 | 132 |

Beispiel 2

| | |
|---|---|
| 200 | 158 |
| 100 | 134 |

Beispiel 3

| | |
|---|---|
| 100 | 300 |
| 50 | 180 |
| 25 | 157 |
| 12,5 | 132 |

...

0072987
HENKEL KGaA
ZR-FE/Patente

## Patentansprüche

1. Neue Glycidyl-substituierte 1,3,2-Oxazaphosphorin-Verbindungen der allgemeinen Formel I

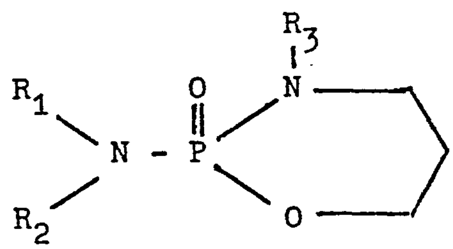

I

in $R_1$, $R_2$ und $R_3$ Wasserstoff, den Glycidylrest und/oder einen 3-Hydroxy-2-Halogen-Propylrest mit der Maßgabe bedeuten, daß wenigstens zwei Glycidylreste oder wenigstens ein Glycidylrest zusammen mit wenigstens einem 3-Hydroxy-2-Halogen-Propyl-Rest vorliegen, wobei $R_1$ auch einen Kohlenwasserstoffrest bedeuten kann.

2. Neue 1,3,2-Oxazaphosphorin-Verbindungen nach Anspruch 1 dadurch gekennzeichnet, daß $R_3$ den Glycidylrest oder einen 3-Hydroxy-2-Halogen-Propyl-Rest bedeutet.

3. Neue 1,3,2-Oxazaphosphorin-Verbindungen nach Ansprüchen 1 und 2 dadurch gekennzeichnet, daß als Halogen im 3-Hydroxy-2-Halogen-Propyl-Rest Brom oder insbesondere Chlor vorliegt.

4. Neue 1,3,2-Oxazaphosphorin-Verbindungen nach Ansprüchen 1 bis 3 dadurch gekennzeichnet, daß der als $R_1$ gegebenenfalls vorliegende Kohlenwasserstoffrest nicht mehr als 12, insbesondere nicht mehr als 6 Kohlenstoffatome aufweist und insbesondere ein niederer Alkylrest ist.

...

5. Arzneimittelzubereitungen mit cytostatischer Wirksamkeit, enthaltend Verbindungen der allgemeinen Formel I insbesondere in Abmischung mit üblichen Hilfs- und Trägerstoffen.

6. Verfahren zur Herstellung der neuen 1,3,2-Oxazaphosphorin-Verbindungen der allgemeinen Formel I dadurch gekennzeichnet, daß man in 1,3,2-Oxazaphosphorin-Verbindungen der allgemeinen Formel II

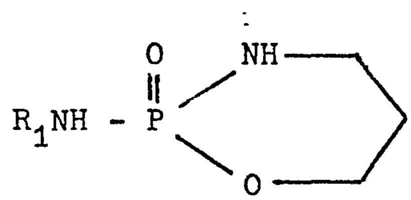 II

in der $R_1$ Wasserstoff oder einen Kohlenwasserstoffrest bedeutet den Glycidylrest der allgemeinen Formel III

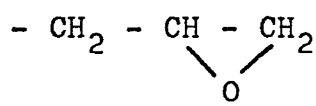 III

und/oder den 3-Hydroxy-2-Halogen-Propyl-Rest in an sich bekannter Weise, insbesondere durch Umsetzung der Verbindungen der allgemeinen Formel II mit einer Epihalohydrin-Verbindung einführt.

Europäisches Patentamt

# EUROPÄISCHER RECHERCHENBERICHT

0072987

Nummer der Anmeldung

EP 82 10 7361

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| D,Y | DE-A-2 907 349 (HENKEL KGaA) * Insgesamt * | 1,5 | C 07 F 9/65<br>A 61 K 31/675 |
| | --- | | |
| D,Y | EP-A-0 033 503 (HENKEL KGaA) * Ansprüche * | 1,5 | |
| | --- | | |
| D,Y | GB-A- 812 651 (ASTA-WERKE AG) * Insgesamt * | 1,5 | |
| | --- | | |
| Y | FR-M- 6 695 (ASTA-WERKE AG) * Insgesamt * | 1,5 | |
| | ----- | | |
| | | | RECHERCHIERTE SACHGEBIETE (Int. Cl. ³) |
| | | | C 07 F 9/00 |

Der vorliegende Recherchenbericht wurde für alle Patentansprüche erstellt.

| Recherchenort | Abschlußdatum der Recherche | Prüfer |
|---|---|---|
| DEN HAAG | 01-10-1982 | BESLIER L.M. |

KATEGORIE DER GENANNTEN DOKUMENTEN

- X : von besonderer Bedeutung allein betrachtet
- Y : von besonderer Bedeutung in Verbindung mit einer anderen Veröffentlichung derselben Kategorie
- A : technologischer Hintergrund
- O : nichtschriftliche Offenbarung
- P : Zwischenliteratur
- T : der Erfindung zugrunde liegende Theorien oder Grundsätze
- E : älteres Patentdokument, das jedoch erst am oder nach dem Anmeldedatum veröffentlicht worden ist
- D : in der Anmeldung angeführtes Dokument
- L : aus andern Gründen angeführtes Dokument
- & : Mitglied der gleichen Patentfamilie, übereinstimmendes Dokument

EPA Form 1503. 03 82